# EUROPEAN PATENT SPECIFICATION

(11) **EP 1 358 852 B1**
(45) Date of publication and mention of the grant of the patent: **14.12.2005**
(21) Application number: 02425279.3
(22) Date of filing: 03.05.2002
(51) Int. Cl.: A61B 17/17

(54) **Apparatus for the osteosynthesis of bone fractures by means of locked endomedullary nailing**
Vorrichtung für die Osteosynthese von Knochenbrüchen mittels Markhöhlennägeln
Appareil pour ostéosynthèse de fragments osseux par clou endomédullaire

(43) Date of publication of application: 05.11.2003
(73) Proprietor: Trinchese, Luciano, 47838 Riccione (Rimini) (IT)
(72) Inventor: Trinchese, Luciano, 47838 Riccione (Rimini) (IT)
(74) Representative: Lanzoni, Luciano

(56) References cited:
- WO-A-97/13467
- WO-A-98/32387
- DE-U- 20 015 775
- US-A- 5 411 503

## Description

The present invention relates to an apparatus for the osteosynthesis of bone fractures by means of locked endomedullary nailing.

Locked endomedullary nailing is used in orthopaedic-traumatological surgery, in particular in the treatment of unstable (or complex or comminuted) diaphyseal fractures of long bones, requiring locked osteosynthesis.

All known apparatuses of this kind comprise an endomedullary nail, usually made of steel. It is generally derived from a tubular model with substantially cylindrical symmetry, coaxially defining a through internal channel for the insertion of the nail itself on a so-called "guide wire".

In proximity to its tip, also known as "distal end", the endomedullary nail has two through holes, also known as "distal holes", obtained transversely to the axis of the nail for the insertion of corresponding distal locking screws (or pins). In proximity to its head, also known as the "proximal end", the nail also has two similar holes, also known as "proximal holes", for the insertion of corresponding proximal locking screws (or pins).

The proximal and/or distal holes have a diameter of a few millimetres. The axes of the proximal and distal holes intersect the axis of the nail. The two distal holes have axes that are mutually parallel and perpendicular to the axis of the nail. The proximal holes can have axes variously oriented relative to each other, depending on the type of nail used and on the fracture type.

Given the tubular nature of the nail, each through hole (be it distal or proximal) is reduced to two coaxial circular openings, obtained on the walls of the nail at opposite sides relative to the axis thereof.

Length, outer diameter and, possibly, diameter of the inner channel of the nail vary depending on the type of fracture to be treated.

Some types of nails (generally, those with proximal holes whose axis are not mutually parallel) can be slightly bent back in correspondence with the proximal end.

When treating a fracture, an end of the bone to be recomposed is surgically exposed and an access opening to the endomedullary channel is obtained therein. A thin steel guide wire (possibly, slightly bent at its tip) is then inserted into the access opening, making the guide wire pass inside the portions of endomedullary channel of all the fragments of the fractured bone. This operation is performed with the aid of an X-ray vision system comprising: an emitter of X-rays that irradiates the area of interest from the outside, a corresponding detector located at the opposite side and provided with brilliance amplifier, a viewing screen.

The endomedullary nail, threaded on the guide wire, is then inserted inside the endomedullary channel of the fractured bone, so as to align on itself all the fractured parts of the bone thanks to the action of the guide wire whereon the nail slides. The nail is inserted into the bone nearly to the proximal end, in such a way that the proximal and distal holes are completely inside the bone. This operation is performed manually, generally with the aid of a transverse grip, also known as "hand piece", which is preventively associated in removable fashion to the proximal end of the nail by means of a fastening pin coaxial to the nail itself. The fastening pin is also provided with an inner channel for the passage of the guide wire. At the end of the insertion of the endomedullary nail, the wire guide is extracted by removing it from the inner channel of the nail.

At this point, the nail must be locked in position by driving the distal and proximal locking screws (or pins) into the bone through the corresponding distal and proximal holes. To obtain the passage for the locking screws, it is necessary to drill the bone from side to side exactly along the axis, respectively, of the proximal holes and of the distal holes (obviously, after surgically uncovering the bone parts in correspondence with which said holes are located). This operation, for which a drilling organ is used (for instance, a drill fitted with a suitable bit), must be performed very carefully.

The problem of driving the locking screws of the nail is very delicate. It is easy to make mistakes in identifying the exact position of the axis of the (proximal or distal) hole of the nail along which the bone must be drilled and, consequently, it is also easy to make mistakes in aligning the drilling organ with the axis of the hole.

A mistake in the bone drilling phase can compromise the stability of the nail implantation and, therefore, the outcome of the surgical operation.

Therefore, usually, known apparatuses for the osteosynthesis of bone fractures by means of locked endomedullary nailing are provided with devices for locating the axis of the hole (distal or proximal) along which the bone must be drilled, which may be provided with accessories for aligning the drilling organ with said axis.

Manual locating devices are known, based on empirical techniques, which provide for the combined use of an X-ray vision system like the one described above and of a simple, thin reference rod, called "Kirschner wire".

After accurately positioning emitter and detector of the viewing system astride the bone, in such a way that the image of the hole of the nail thus obtained is perfectly circular, the surgeon tries to place the sharp tip of the Kirschner wire in correspondence with the centre of the image of the hole. Once this position is found, keeping the sharp tip still, the Kirschner wire is oriented in such a way that its image, produced by the viewing system, is substantially reduced to a point located approximately in the centre of the image of the hole. In this way, the Kirschner wire and the axis of the hole are approximately aligned. The Kirschner wire is driven slightly into the cortical of the bone, in such a way as to clearly indicate driving point and direction of drilling. Generally, at this point the Kirschner wire is removed and, exploiting the small incision produced thereby as a reference for positioning the drill bit, the perforation is made after trying to position the axis of the drill bit correctly. In performing this latter operation it is very easy to make alignment mistakes, so an alternative solution is not to remove the Kirschner wire and to use it as a physical reference for a tubular drill bit that can be inserted directly on the wire itself used as a guide.

This solution to the alignment problem has very numerous disadvantages, which can be summarised as follows in order of severity.

First of all, the search for the driving point of the locking screws, completely left to the surgeon's skill and manual ability, is complicated and does not guarantee the final result. All operations (positioning the end of the Kirschner wire in correspondence with the hole and subsequent optimisation of the relative position of both, also to determine the direction of the axis of the hole correctly and to allow subsequent alignment of the drilling organ therewith) are performed exposing the treated part to X-rays, whilst the surgeon is forced to monitor the operations through a screen and not by directly viewing them. The times required to install the locking screws are extremely variable in relation to several factors, such as: the skill of the surgeon and of the radiologist, the morphology of the patient and the proper positioning of the X-ray viewing system relative to the part to be treated. Moreover, both the patient and the medical personnel are exposed to a high dose of dangerous ionising radiation during the entire nail locking phase. The time required by involved medical personnel to learn this technique is very long, as it requires a considerable level of manual skill and experience. The complex and delicate nature of the operations to be performed entails a high risk of error.

A recent, modern technique employs appropriate radiographic spatial references which, once the nail is inserted into the endomedullary channel and before the bone is drilled, are placed in various positions of the bone segment to be treated. Subsequently, a series of radiographic images of the bone segment to be treated is taken from various positions and a virtual reality, based on such images, is reconstructed, allowing the surgeon to drill the holes and to position the locking screws of the nail.

This solution to the problem, however, is not free from drawbacks.

Very costly equipment is required, which makes use of said techniques and devices possible practically only in large hospital facilities, which can count on such a number of patients as to allow, in relatively short times, to amortise the acquisition and installation costs of the equipment.

The execution of the operation is in any case complicated and rather laborious. Once again, the time required by medical personnel to learn the technique is long. Moreover, in this case as well, both the patient and the medical personnel are exposed to dangerous ionising radiation during the nail locking phase, albeit to a reduced extent relative to the empirical techniques described above.

Also known are locating devices that use electronic sensors which, made to slide externally on the surface of the bone, are able to detect the difference in impedance due to the characteristics of the endomedullary nail inserted therein. These devices, rarely used today, are not very reliable and they yield occasionally random results. The system is laborious, requires long learning times and has high costs.

Also known are locating devices that use mechanical references of various kinds (sometimes made with materials that are transparent to X-rays) which, positioned externally to the bone, can be made integral with the proximal end of the nail and serve as supports to guides for the bit of the drilling organ and/or for the locking screws. If the mechanical references are positioned correctly relative to the proximal end of the nail, the positions in which the guides are located are, in principle, aligned with those of the proximal and/or distal holes of the nail. The mechanical references are provided with graduated gauges that allow for their correct positioning depending on the length of the nail.

An example of commonly used mechanical reference is a graduated bar that can be made integral with the proximal end of the nail and whereto various accessories can be associated.

Alignment precision thus depends to a considerable extent on the rigidity of the nail-reference system. This rigidity is never absolute, because of the play linked to the coupling of the various mechanical parts and of the intrinsic elasticity of the nail. The nail can flex by effect of the torsion and flexion forces exerted by the mechanical systems used to place the fractured part under traction and/or by effect of the tensions exerted by the soft parts on the nail-reference system.

This problem is aggravated by the fact that the mechanical reference, developing parallel to the axis of the nail, is not fastened in any way to the distal end of the nail itself and thus has a free end. The nail-reference system is therefore "open" in correspondence with the distal end. Near the proximal end, it is sufficiently rigid to minimise deformation and thus assure a sufficient precision in the alignment of the drilling organ with the proximal holes, but it is never so rigid as to prevent, progressively farther away from the proximal end, the relative deformation of the two elements from becoming such as to render uncertain the correspondence between the guides and the holes on the nail. Thus, the device has high probabilities of failure in the driving of the distal screws.

To overcome this problem, the surgeon tries to close the nail-reference system in correspondence with the distal end of the nail by means of a stabiliser rod. The stabiliser rod is mounted at the free end of the mechanical reference in such a way as to be perpendicular to the plane defined by the mutually parallel axes of the distal holes of the nail. It must be brought manually in contact with the proximal end of the nail in correspondence with the axis of the nail itself and maintained there, also manually, during the drilling and distal screw insertion operations.

This solution solves only partially the problem of stiffening the system and it is very complicated, as it requires the intervention of another person, in addition to the surgeon, to keep the stabiliser rod in contact with the nail. Moreover, the stabiliser rod must be partially inserted inside the bone in a (obviously, after surgically uncovering this additional part of bone as well) it is necessary to execute an additional partial perforation of the bone perpendicularly to the plane defined by the axes of the distal hole, after correctly aligning the bit of the drilling organ.

In the second place, the use of locating devices provided with mechanical references does not completely eliminate the need to monitor operations using the X-ray vision system. Hence, although to a lesser extent than in the previous techniques, patient and medical personnel are still exposed to dangerous ionising radiation during the nail locking operation.

Moreover, said devices require a complex and costly surgical instrumentation, with consequent difficulties with its use, maintenance, sterilisation and storage.

In general, currently used systems are therefore burdened, depending on the case, by an excessive complexity (often accompanied by a certain degree of imprecision) and/or by an inevitable, and excessive, exposure of the patient and of medical personnel to dangerous radiation sources.

It is also known from Document US 5411503 an instrumentation for distal targeting of locking screws in intramedullary nails which comprises a probe inserted into the intramedullary nail. Said probe positions two electromagnetic drive coils with their magnetic axes parallel to and at a fixed offset from the axis of a transverse hole to be drilled.

The drive coils generate alternating magnetic fields over intermittent and nonoverlapping time intervals in a manner that provides independent sources of positional information.

A hand-held guide, containing a drill brushing and four receiving coils with the same offset as the drive coils, when moved about in the vicinity of the probe, produces a corresponding movement of graphical images on a display screen which helps the surgeon while making a stab incision to the bone and while inserting and fastening the locking screw.

The aim of the present invention is to overcome the above drawbacks, making available an apparatus for the reduction of bone fractures by means of locked endomedullary nailing, which allows simply and without excessive exposure to dangerous radiation to locate with certainty the holes for the insertion of the locking screws.

Another aim of the present invention is to make available an apparatus for the reduction of bone fractures by means of locked endomedullary nailing, which allows to nimimise the time required to execute the nail locking operations, whilst assuring a successful outcome. operations, whilst assuring a successful outcome.

These aims and others besides, which shall become more readily apparent from the description that follows, are achieved, in accordance with the present invention, by an apparatus for the reduction of bone fractures by means of locked endomedullary nailing as described and claimed in the accompanying main claim and in the accompanying dependent claims.

The invention is disclosed in greater detail below with the aid of the drawings, which show an embodiment provided purely by way of non limiting example.
- Figure 1 shows a schematic perspective view of a bone with the apparatus of the invention applied thereto.
- Figure 2 shows, partially sectioned in a plane containing the axis of the bone and the axis of a hole, a variation of the apparatus of Figure 1, with the emitter in working position.
- Figure 3 shows a cross section of the bone and of the nail with a detail of a variation of the apparatus of the invention.
- Figures 4a, 4b, 4c show an operating principle of the invention together with an additional variation thereof.
- Figure 5 shows, partially sectioned in the plane of Figure 2, a variation of the invention with the emitter in working position.
- Figure 6 shows, partially sectioned in the plane of Figure 2, a detail of a variation of emitter in working position.
- Figures 7 e 8 respectively show a partial section in the plane of Figure 2 and in a plane perpendicular thereto of an additional variation of the invention with the emitter in working position.
- Figure 9 shows an exploded view of the assembly comprising a nail, a hand piece and a corresponding coaxial fastening pin.
- Figures 10a and 10b show a longitudinal section of the coupling area between the nail, hand piece and fastening pin respectively with the pieces separate and with the pieces mutually joined by the fastening pin.
- Figures 11a and 11b show a cross section of the variation of Figures 7 and 8 with the emitter, respectively, away from the working position and in working position.

With reference to the figures, an apparatus for the osteosynthesis of bone fractures by means of locked endomedullary nailing comprises a tubular nail 1, which defines a through coaxial internal channel 2. The nail 1, provided with transverse through holes 5a, 5b, 5c, 5d whose axis 6a, 6b, 6c, 6d intersects the axis 7 of the nail 1, can be inserted in a medullar channel 3 of a bone 4. The apparatus further comprises a device for locating the axis 6a of a predetermined hole 5a selected among said holes 5a, 5b, 5c, 5d, axis 6a along which the bone 4 is to be drilled to drive a corresponding screw (not shown herein) for locking the nail 1 on the bone 4.

The locating device comprises a source 8 of electromagnetic power, an emitter 9 of the electromagnetic power in the form of non ionising electromagnetic radiation and a line 15 for transmitting the electromagnetic power from the source 8 to the emitter 9.

The emitter 9 of the electromagnetic power in the form of non ionising electromagnetic radiation can be inserted inside the nail 1 along the internal channel 2. Its travel along the internal channel 2 extends from a proximal end 10 of the nail 1 at least to its work position, located on the axis 6a of the predetermined hole 5a, in which at least part of the non ionising electromagnetic radiation is directed from the emitter 9, through the predetermined hole 5a, on an inner superficial portion 11 of the cortex 12 of the bone 4 corresponding to the axis 6a of the predetermined hole 5a and generates, beyond an outer superficial portion 13 of the cortex 12 of the bone 4, also corresponding to the axis 6a of the predetermined hole 5a, a signal 14 detectable from the exterior and having an intensity distribution with its centroid in correspondence with the axis 6a of the predetermined hole 5a. Obviously, also the transmission line 15 can be inserted at least partially along the internal channel 2, to follow the travel of the emitter 9.

Since the nail 1 is substantially opaque to the radiation exiting the emitter 9, whilst bone tissues are traversed by it (though they may be involved in diffusion and absorption phenomena by the electromagnetic radiation that propagates therein), the signal 14 is generated beyond the outer superficial portion 13 only when the emitter 9 itself is in front of the predetermined hole 5a (or of one of the other holes, be they distal or proximal, into which the locking screws are to be inserted). Since the signal 14 can be detected from the exterior, the hole itself makes itself "visible" to the surgeon. Since the radiation is not ionising, no person present in the operating room is subjected to harmful radiation loads and the surgeon can take the time needed to adjust the position of the emitter 9 exactly on the work position without particular problems (compatibly with the patient's anaesthesia time).

Opportunely, the source 8 has its power adjustable in order to adjust at will (at least within certain limits) the intensity of the signal 14 detectable from the exterior.

In general (except for a variation described below, which constitutes an exception), the signal 14 is essentially due to that part of electromagnetic radiation, exiting the emitter 9, that is transmitted through the bone tissues. When the non ionising electromagnetic radiation, exiting the emitter 9, has a spectrum distributed on a predetermined interval of wavelength selected in the range that goes from the near ultraviolet up to and including the mid infrared, the signal 14 is essentially determined by that fraction of electromagnetic radiation that, after exiting the emitter 9, traversed the cortex 12 of the bone 4 after undergoing absorption and/or diffusion phenomena by the bone tissues, the liquids and the soft parts encountered in its path from the emitter 9 towards the exterior. When the electromagnetic radiation exiting the emitter 9 is a part of the thermal infrared range, the transmitted radiation that determines the signal 14 has a partly different origin. The same liquids, soft parts and bone tissues, heated by the thermal radiation received from the emitter 9, in turn become sources of thermal radiation that is partly emitted towards the exterior, so that the intensity distribution of the signal 14 detectable from the exterior depends not only on absorption and diffusion phenomena, but also on phenomena of heat transfer in biological tissues.

In both cases, anyway, the operating principle is based on an appropriate illumination of the predetermined hole 5a from the interior of the nail 1 with a beam of electromagnetic radiation (at long, i.e. non ionising, waves), and on the detection of the radiation transmitted through the bone tissue in an area surrounding the incidence point. The point in which the bone 4 is to be drilled for the insertion of the locking screw coincides with the centroid of the distribution of the transmitted radiation.

Advantageously, the emitter 9 can be structured in such a way that the centroid of the intensity distribution of the signal 14 detectable from the exterior coincides with an intensity peak 140, so that the point where the bone 4 is to be drilled for the insertion of the locking screw coincides with the maximum of the distribution of the transmitted radiation.

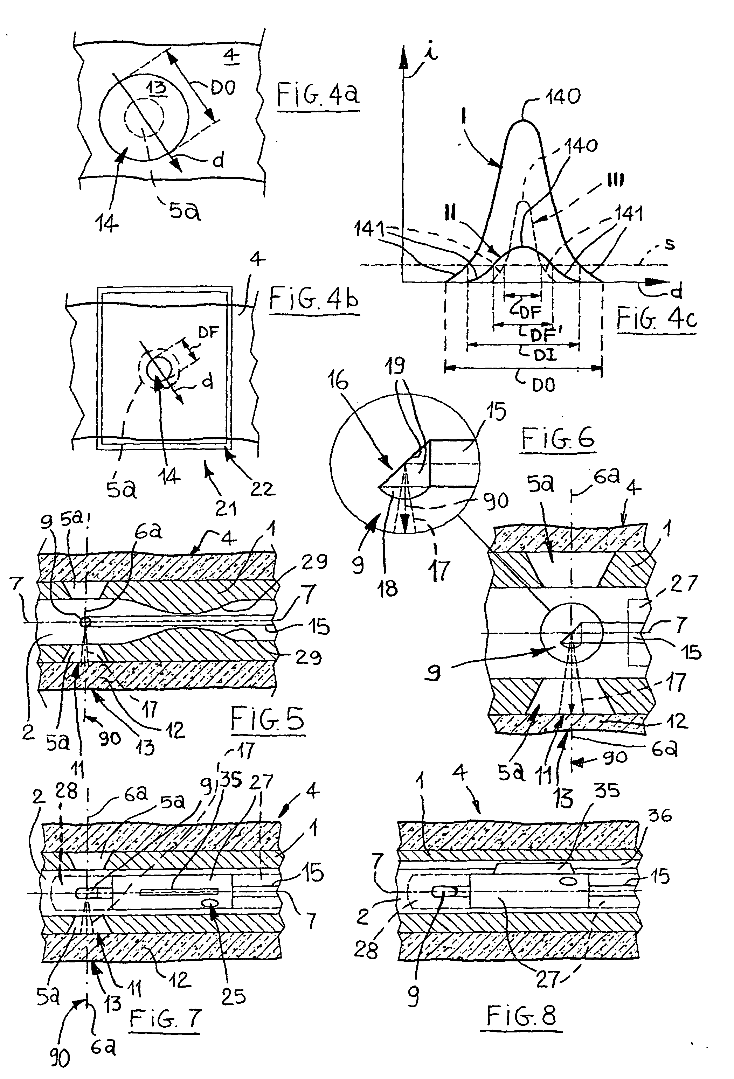

In particular, opportunely, in a preferred embodiment of the invention illustrated in Figure 6, the emitter 9 comprises an optical collimation system 16 that collimates the electromagnetic radiation in a beam within a predetermined solid angle 17 centred on the optical system 16, the solid angle 17 being coaxial with the predetermined hole 5a when the emitter 9 is in the work position.

When, as generally occurs (and is preferable in many cases), the source 8 generates the non ionising electromagnetic radiation and the emitter 9 serves merely as a conduit, the transmission line 15 comprises a waveguide for transporting the electromagnetic radiation to the emitter 9.

If the emitter 9 also comprises the optical collimation system 16, then the latter is directly coupled to the waveguide. In this case, the optical system 16, coupled to the waveguide, conveniently comprises a collimating lens 18 and a deflector 19 that deflects the radiation coming from the waveguide in the solid angle 17. The deflector 19 can be a mirror (for instance inclined by 45° relative to the axis of the waveguide), or a prism with total internal reflection. The presence of the deflector 19 is generally opportune to diminish the risk that, as a result of a marked bending of the waveguide when the emitter 9 is oriented correctly in view of its insertion into the internal channel 2, there is either a heavy loss of intensity of the radiation exiting the emitter 9 or a breakage of the waveguide itself.

In general, the waveguide can comprise an optical fibre. This optical fibre can be either single-mode or multi-mode. The waveguide can also comprise a fibre bundle, depending on requirements. Given the low powers generally involved (with the exception constituted by the variation described farther on, wherein high powers are involved), optical fibres made of plastic material can also be used.

It is highly opportune to collimate the radiation exiting the emitter 9 when it is distributed on wavelengths for which propagation occurs only by direct irradiation, such as wavelengths belonging to the ultraviolet, visible or near infrared ranges. The bone tissue is diffusing. Thus generally, even when the emitter 9 comprises the optical collimation system 16, the radiation transmitted beyond the cortex 12 of the bone 4 ends up having a substantially Lambertian angular distribution. Starting from an emitter 9 from which egresses a radiation beam corresponding to a non negligible solid angle 17 (for instance that of a non collimated radiation), there is a marked (and, at the limit, an unacceptable) decrease in contrast due to attenuation by diffusion.

The apparatus can be provided with a detector 24 of the signal 14 and with a related image converter, which can be positioned externally to the bone 4, to identify the centroid of the intensity distribution of the signal 14 and to display its position. This viewing system can comprise an image converter, a television camera (visible or infrared), a photodiode or light detector system. It can comprise light multipliers and/or image intensifiers, or any optoelectronic system able to make visible and/or intensify or attenuate or appropriately filter the electromagnetic radiation transmitted through the bone tissue.

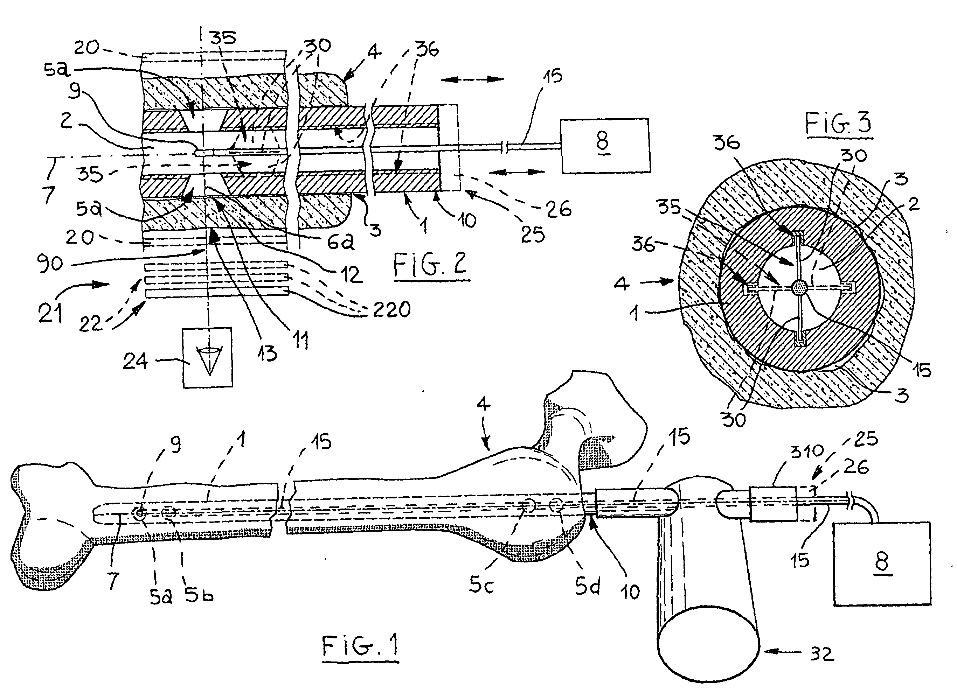

Advantageously, however, if the radiation exiting the emitter 9 is distributed over wavelengths belonging to the range of the visible spectrum (i.e. essentially between 380 nanometres and 750 nanometres), the surgeon can identify the centroid of the intensity distribution of the signal 14 by direct vision (possibility indicated by the eye symbol shown in Figure 2), without any other kind of aid. This advantage can lead to favour the choice of a system that uses visible radiation rather than one that uses infrared radiation which, though less prone to diffusion by the traversed tissues, makes it necessary to use auxiliary viewing systems.

If the source 8 directly generates electromagnetic radiation, subsequently carried to the emitter 9 by a waveguide, the source 8 can be a common source of visible light of appropriate spectrum (or according to its own characteristic spectrum), for instance incandescence, or gas discharge, or halogen. In this case, the sources are generally broad band sources.

The emission spectrum of the source 8 can also be modified by placing in front of it appropriate chromatic filters that select a predetermined portion of the visible wavelength range.

Alternatively, it is possible to use narrow band sources, such as lasers or LEDs, either with continuous wave or pulsed (in this case, to increase the peak emission power), sources that can be produced with variable emission wavelength, according to the type of laser or LED, from near ultraviolet to near infrared (as well as mid infrared in the case of some lasers). It is easy to find, for instance, low power lasers, which are coupled well with single-mode optical fibres, which emit powers in the order of 5 mW in the visible range or 10 mW in the near infrared range (for instance on wavelengths in the order of about 1 micrometre).

Very widespread (and particularly useful, as we shall see) are low power He-Ne lasers, with emission wavelength in the red range, around 633 nanometres, which need only to be coupled to the waveguide or optical fibre. There are also (low power) semiconductor lasers (with wavelengths also in the red range) already coupled to one or more optical fibres. They are very simple and economical systems. There are semiconductor lasers with emission peak practically on any useful wavelength.

It is particularly advantageous for the electromagnetic radiation exiting the emitter 9 to have its spectrum distributed over visible wavelengths ranging between 600 nanometres and 700 nanometres.

The absorption characteristics of the tissues and of the liquids which the electromagnetic radiation itself must traverse in its path from the emitter 9 to the exterior of the bone 4 have a great influence on the generation of the signal 14 on the outer superficial portion 13 of the cortex 12 of the bone 4 and on its intensity.

The interactions of blood, of the liquids inside the endomedullary channel 3, of the soft tissues (and, in general, of every component in the endomedullary channel 3 and/or in the bone 4) with the various radiations, according to their wavelength, are very important.

It is necessary to consider the absorption spectrum of blood and/or of biological tissues in general (which is not flat, but has areas of greater absorption and a transmission/transparency window for wavelengths centred around red) combined with the spectrum of the bone and of the tissues.

In general, the main causes of absorption in biological tissues are due to: oxy-haemoglobin (present in arterial blood); deoxy-haemoglobin (present in venous blood); water and various liquids; lipids. Within bone tissues, things change a little due to the contribution and the presence of collagen and of other bone structures. The sum of all these effects produces a global absorption spectrum that exhibits, on average, a first minimum between 600 and 700 nanometres, which gives rise to a dark red colour, and a second minimum around 800 nanometres. For wavelengths of less than 600 nanometres (towards green), absorption tends to grow, as it does for wavelengths exceeding 800 nanometres. Around 900 nanometres, there is a new absorption peak mainly due to the presence of water.

This is why it is very advantageous to use a source 8 that emits visible light on wavelengths of between 600 and 700 nanometres (or a broad band source 8 appropriately provided, as it egresses towards the waveguide, with spectrally selective filters on this wavelength range).

Ideal sources for the present purposes are: He-Ne lasers (emission on wavelengths near 633 nanometres) or semiconductor lasers calibrated on red. In addition to being optimal from the spectral viewpoint, these sources exhibit a low scattering level and, hence, allow to attain an excellent compromise on the level of attenuation of the radiation by diffusion and absorption on the part of the traversed tissues.

To facilitate the identification of the centroid of the intensity distribution of the signal 14 detectable from the exterior, in particular when it coincides with the intensity peak 140, the apparatus of the invention further comprises a contrast enhancing device 21, which can be positioned on the axis 6a of the predetermined hole 5a externally to the bone 4 and acts on the radiation portion transmitted through the bone tissues, enhancing the contrast between the intensity peak 140 and a peripheral part 141 of the signal 13 and improving resolution in the identification of the position of the axis 6a on the predetermined hole 5a.

Opportunely the contrast enhancing device 21 comprises an attennuating filter 22 for attenuating the radiation intensity. Said attenuating filter 22 lets through only the central, generally more intense, part of the distribution of the signal intensity 14, enhancing the contrast factor and the resolution.

The attenuation factor of the attenuating filter 22 can be defined as the ratio of the intensity of the light incident on the attenuating filter 22 and the intensity of the light transmitted thereby. It can be defined as a spectral parameter, i.e. calculated at each individual wavelength, or as an integral parameter, i.e. defined relative to total intensity (over all wavelengths together) of the radiation. Opportunely, said attenuation factor can be made adjustable discretely (for instance by superposing in removable fashion multiple attenuating elements 220 to constitute a single attenuating filter 22, as shown schematically in Figure 2), or continuously (for instance, using special filters obtained with electrochromic materials), to obtain an optimal contrast factor.

The contrast enhancing device 21 can be integrated in the detector 24 of the signal 14. In the preferred variation described herein, i.e. the one that uses visible radiation, since the signal is directly observable by the operator, it is advantageously possible to dispense with the detector 24 and the systems connected thereto. In this case the contrast enhancing device 21 can be integrated in a simple pair of eyeglasses, wearable by the surgeon, provided with seats for the insertion of one or more attenuating filters 22.

A principle according to which the use of such an attenuating filter 22 enhances the contrast between the intensity peak 140 and the peripheral part 141 of the signal 14 is schematically illustrated in Figures 4a, 4b, 4c (in which the represented components are illustrated only for purposes of explaining the principle, without regard to the actual dimensions and proportions).

The original distribution of the electromagnetic radiation which, egressing from the emitter 9, passes through the predetermined hole 5a, is modified traversing the cortex 12 of the bone 4 as a result of the multiple diffusion processes undergone through the bone tissue (and as a result of the absorption due mainly to liquids and soft tissues). When the radiation itself reaches the outer superficial portion 13 of the cortex 12, its distribution, as shown in Figure 4a, is broadened over an area that as a first approximation has generally circular symmetry and has a diameter D0. In Figure 4a said diameter D0 is shown greater than the diameter of the predetermined hole 5a (which, depending on the emission characteristics of the emitter 9, can frequently be the case). Moving on the outer superficial portion 13 along a straight line d superposed to a diameter of the approximately circular area whereon the signal 14 is distributed, the intensity i of the signal 14 varies. Its profile is generally the one schematically illustrated by the curve indicated as I in Figure 4c.

Since the eye is easily saturated above a certain intensity threshold (indicated with the straight line s in Figure 4c), the broadened circular area has an ample central area with diameter DI which is perceived as uniformly illuminated and whose centre is not identified. Attenuating the signal 14, depending on the characteristics of the attenuating filter 22, the intensity distribution, originally represented by the curve indicated as I, can be transformed for example into a distribution like those represented respectively by the curve indicated as II and by the curve indicated as III in Figure 4c. The central area, perceived as uniform, is greatly reduced in diameter (as shown in Figure 4c by the indication of the diameters DF and DF', as well as in Figure 4b, which illustrates the application of an attenuating filter 22 and its effect, compared to the initial configuration illustrated in Figure 4a). Using appropriate attenuating filters 22 it is possible to reduce the diameter of the central area to the point of making visible only a small area corresponding to the axis 6a of the predetermined hole 5a wherefrom the light comes.

The attenuating filter 22 can comprise an attenuating element 220 of the "neutral density" type (i.e. with transmission spectrum substantially constant as a function of the wavelength) in the visible range. In combination with He-Ne lasers, attenuation factors between 1000 and 10000 (achievable with a single filter or with a combination of filters) yield good results.

To try to exploit the mechanisms of attenuation by absorption of the materials traversed by the radiation (and which have already been discussed in relation to the spectral characteristics of the radiation egressing from the emitter 9), it is also possible to use a spectrally profiled filter which allows the passage of the red radiation which (as stated also in relation to the sources) undergoes less diffusion/attenuation, allowing further to enhance contrast. In particular, the attenuating filter 22 can comprise an attenuating element 220 that attenuates the transmitted radiation whose wavelength is less than 600 nm. Preferably, the transmission spectrum of the attenuating element 220 has a maximum for wavelengths between 600 nm and 700 nm. In regard to this issue, it is necessary to note the importance of optimising both the spectral selectivity of the filter and (as the case may warrant) that of the emission spectrum of the source according to the absorption characteristics of the tissues and of the liquids which the radiation is to traverse.

Using, even with different sources, filters with spectrally selective transmission in these regions of the visible spectrum allows an additional, marked enhancement of contrast, eliminating all those components that are attenuated by the tissues and that only saturate the sensitivity of the eye.

Obviously, combinations of different types of attenuating elements 220 can be used (both "neutral density" and spectrally profiled, also with mutually different transmission spectra).

In a particular variation of the invention, opportunely, from the emitter 9 egresses electromagnetic radiation with a determined polarisation state and the attenuating filter 22 comprises a polariser element to attenuate to the highest possible extent the portion of radiation transmitted by the bone tissues not having the polarisation state of the radiation originally egressing from the emitter 9.

The diffusion of the electromagnetic radiation due mainly to bone tissues has a depolarising effect on the transmitted electromagnetic radiation. From the centre of the illuminated circular area, which is formed on the outer superficial portion 13 of the cortex 12 of the bone 4, emerges, towards the attenuating filter 22 the light that is most likely to reach the eye after traversing the bone without severe interference. Its polarisation state is generally very similar to the original one, whilst the light emerging from the periphery of the same area tends to be almost totally depolarised. The contrast enhancement is achieved, in fact, by highlighting the light that, having a very similar polarisation state to the original one, did not diffuse and, hence, is still collimated around the axis 6a of the predetermined hole 5a. The polarised light can be emitted directly by the source 8 or be obtained therefrom by placing an appropriate polariser filter between the source 8 and the waveguide.

Advantageously, provisions can be made to minimise the time required to adjust the position of the emitter 9 exactly on the working position, thereby reducing the probability of errors and the execution time (with the consequent reduction of the quantities of anaesthetic to be administered to the patient).

In particular, the apparatus according to the invention can comprise automatic positioning means for automatically positioning the emitter 9 at the height of the plane defined by the axis 6a of the predetermined hole 5a and by the axis 7 of the nail 1, acting at least when the emitter 9, inserted in the internal channel 2, is in proximity to the working position.

It is not strictly necessary for the emitter 9 also to be on the axis 7 of the nail 1, although it may be advantageous for it to be so in some cases. It is important that the emitter 9 be guided. Obviously, the emitter 9 itself could have dimensions corresponding to the diameter of the internal channel 2, in order to be positioned automatically, but normally the emitter 9 can be small or with a geometry not quite suitable to the purpose.

Advantageously, the automatic positioning means comprise means for centring the emitter 9 on the axis 7 of the nail 1, acting at least when the emitter 9, inserted in the internal channel 2, is in proximity to the working position.

In a first variation of the invention, illustrated in Figures 7, 8, 11a, 11b, said means for centring the emitter 9 on the axis 7 of the nail 1 comprise at least a widening 27 of the diameter of a sheath of the transmission line 15 at least in proximity to the emitter 9.

Opportunely, the widening 27 can be extended to enclose the emitter 9 with an element 28 that is transparent to the electromagnetic radiation egressing from the emitter 9 itself at least in the direction of the predetermined hole 5a. In this way, in addition to serving to protect the emitter 9, the transparent element 28 determines a minimisation of the thickness of potentially absorbing material that the electromagnetic radiation egressing from the emitter 9 must traverse.

In an alternative embodiment illustrated in Figure 5, the means for centring the emitter 9 on the axis 7 of the nail 1 comprise, at least in proximity to the predetermined hole 5a, a narrowing 29 of the internal channel 2 around the axis 7 of the hole 1. Obviously, this solution can be adopted when sheath and emitter 9 have smaller diameter than that of the internal channel 2. Moreover, the narrowing 29 must have a minimum diameter at least corresponding to that of the guide wire, to allow the passage of the nail 1 on the guide wire and the subsequent extraction of the guide wire.

The presence of the narrowing 29 at least for a segment next to the holes 5a, 5b, 5c, 5d (at least next to the distal ones 5a, 5b, for which the present invention is particularly effective, if not next to all of them) prevents warping and allows a self-centering of the system on the axis 7 of the nail 1.

This solution is especially useful for tibial nails 1, that have a curved head (whereas femoral nails are generally straight).

In an additional variation, shown in Figures 1 and 5, the means for centring the emitter 9 on the axis 7 of the nail 1 comprise at least two straps 30 oriented radially relative to the transmission line 15 and positioned along it in proximity to the emitter 9. Preferably, the straps 30 are at the same height along the transmission line 15. If there are more than two, they can be positioned on the various radii egressing from the transmission line 15.

To allow the exact, automatic identification of the working position along the axis 7 of the nail 1, the apparatus of the invention comprises an arrest reference 25 for stopping the insertion of the emitter 9 in the internal channel 2 when the emitter 9 reaches the working position. In combination with the centring means, the arrest reference 25 stops the insertion 2 of the emitter 9 and of a part of the transmission line 15 connected thereto into the internal channel exactly when the emitter 9 is positioned at the centre of the predetermined hole 5a.

At this point, in order for the emitter 9 to be correctly positioned in the working position it is sufficient to orient it in such a way that its emission axis 90 is parallel to the axis 6a of the predetermined hole 5a (if the emitter comprises the optical collimation system 16, said emission axis 90 coincides with the axis of the solid angle 17). For this purpose it may be sufficient to rotate the emitter 9 by acting on the sheath of the transmission line 15.

This can be made possible, for example, by providing at least the terminal segment of the transmission line 15 destined to be inserted into the internal channel 2 of a support element with high torsion constant. Said support element can be incorporated in the sheath or coincide therewith and be made of appropriate material, such as Kevlar. At least the terminal segment of the transmission line 15 destined to be inserted in the internal channel 2 can be enclosed by a substantially rigid sheath (for instance made of Kevlar or steel). At the end of the sheath can be positioned the emitter 9, possibly provided with the optical collimation system 16. It would be opportune for the diameter of the sheath to correspond with that of a normal guide wire.

In general, however, it may not be wholly easy to "seek" the correct orientation of the emitter 9 by twisting the sheath acting on its end accessible by the surgeon's hand (especially if an optical fibre and a flexible sheath are present), to attempt to make it rotate inside the internal channel 2. For the sake of practicality and of operating speed, it is absolutely preferable for the apparatus to comprise means for orienting the emitter 9 with certainty, acting in such a way that its axis of emission 90 is brought parallel to the axis 6a of the predetermined hole 5a. In particular, as shown in the figures, advantageously the apparatus of the invention can comprise means 34 of mutual interference between the terminal segment of the transmission line 15, destined to be inserted in the internal channel 2, and the wall of the internal channel 2 which means, by allowing the transmission line 15 to slide in the internal channel 5, determine the certain orientation of the emitter 9 with its axis of transmission 90 parallel to the axis 6a of the predetermined hole 5a at least in proximity thereto. In a possible embodiment, shown in the figures, the mutual interference means 34 comprise a fin 35 positioned on the transmission line 15 in proximity to the emitter 9 and able to be inserted in sliding fashion at least with one of its ends in a corresponding guiding groove 36 obtained on the surface of the internal channel 2.

More than one fin 35 can be provided. In this case the fins 35 can also coincide with the straps 30 for centring on the axis 7 of the nail 1.

Opportunely, in an embodiment of the invention, the arrest reference 25 comprises stopping elements 33 for stopping the motion of the transmission line 15 in the nail 1, when the emitter 9 reaches the working position, acting between the transmission line 15 and the wall of the internal channel and able to be elastically disengaged, in particular to allow a further motion of the emitter 9 towards a different working position corresponding to another one of the transverse through holes 5a, 5b, 5c, 5d or to extract the emitter 9 from the nail 1. The stopping elements 33 can comprise an expandable head positioned on the emitter 9, destined to be inserted into a seat opportunely obtained in the wall of the internal channel 2.

Alternatively or in combination, the arrest reference 25 comprises an abutment element 26, able to abut the proximal end 10 of the nail 1 or its extension 100 and able to be positioned along the transmission line 15 in a position of arrest of the insertion of the transmission line 15 in the internal channel 2 corresponding to the working position of the emitter 9.

In general, the position of the abutment element 26 can obviously be adjusted according to the hole 5a, 5b, 5c, 5d to be reached and to the length of the nail 1. It can be made coaxial, in sliding fashion, to the sheath of the transmission line 15.

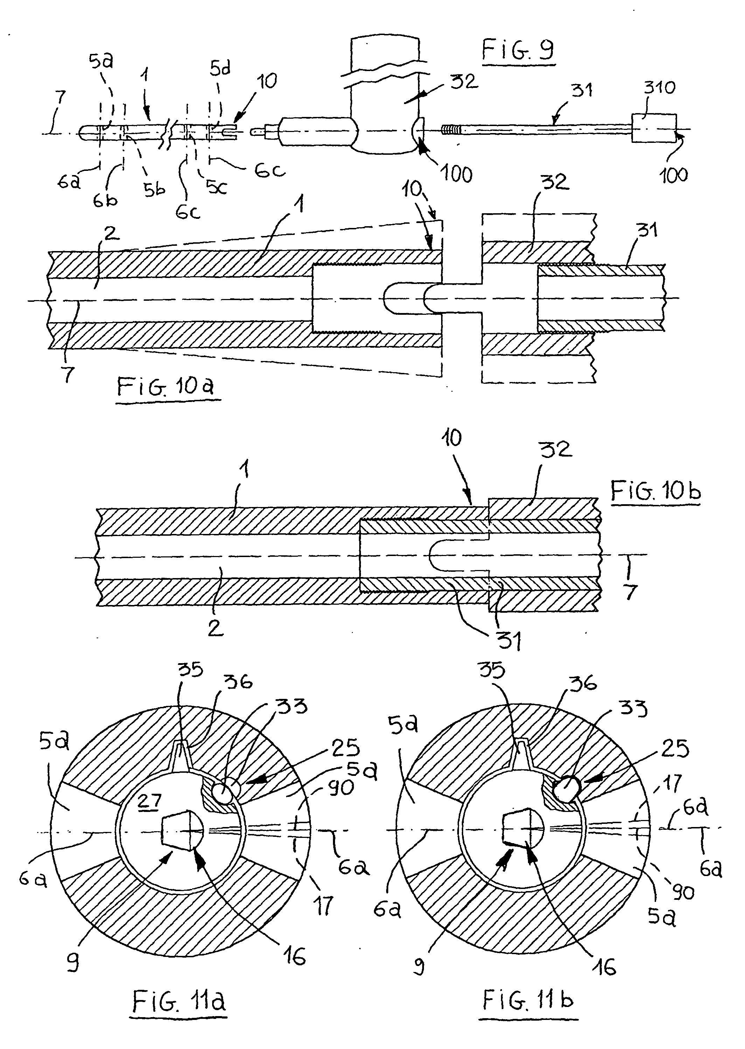

The extension 100 can be a part of a hand piece 32, opportunely associated to the nail 1 in correspondence with the proximal end 10, said part having a passage channel for the emitter 9 and for the corresponding transmission line 15 communicating with the internal channel 2. Alternatively, the extension 100 can also be the head 310 of a fastening pin 31, also tubular to allow the passage of the emitter 9 and of the corresponding transmission line 15, able to be coaxially associated to the nail 1 (and to the hand piece 32, if present) in a known manner. Obviously, as is well known, the hand piece 32 and the corresponding fastening pin 31 must have an inner diameter which allows the passage of at least a guide wire. In partucular, preferably, the apparatus of the invention comprises a fastening pin 31 for fastening a tubular hand piece 32 to the nail 1, the fastening pin 31 being tubular, coaxial to the nail 1 and with its inner diameter corresponding to the diameter of the internal channel 2 of the nail 1. In this latter case, especially in case of nails 1 with small diameter, it may be necessary, to allow the insertion of the fastening pin 31, to have a gradual widening of the nail 1 in proximity to its proximal end 10 (see the dashed line in Figure 10a). These characteristics are illustrated in Figures 9, 10a and 10b.

Use of the abutment element 26 is particularly suitable when the sheath is rigid. In an embodiment of the invention, at least the terminal segment of the transmission line 15 destined to be inserted in the internal channel 2 is enclosed by a substantially rigid sheath and the abutment element 26 and the proximal end 10 of the nail 1 or its extension 100 comprise mutual engagement means for the certain orientation of the emitter 9 with its axis of emission 90 parallel to the axis 6a of the predetermined hole 5a.

Said mutual engagement means can be of various kinds, for instance pins and corresponding seats, or appropriate contoured abutments.

In an embodiment of the invention, the nail 1 comprises, already integrated, emitters 9, already opportunely positioned and aligned at least in front of corresponding distal holes 5a, 5b (optionally also in front of proximal holes 5c and 5d) and provided with corresponding segments of transmission line 15 also integrated in the nail 1 and provided, at their opposite end relative to the emitters 9, with connectors to corresponding extensions of the transmission line 15 to the source 8. The system can thus be readied before inserting the nail 1 on the guide wire in the endomedullary channel 3, integrating all necessary elements in its interior. The nail 1 could also be obtained with single-use integrated elements.

This solution would eliminate every problem of centring, aligning and orienting the emitter 9 relative to the selected hole, since all would already be pre-set.

In a variation of the invention, which can also comprise all (or only some) of the positioning, centring and aligning systems discussed heretofore, the source 8 comprises an electrical power generator, the emitter 9 comprises a heating element and the transmission line 15 comprises an electrical wire connecting the heating element with the electrical power generator, the heating element emitting, when excited by the electrical power, electromagnetic radiation at wavelengths in the thermal infrared region.

The source 8 can be able to provide current continuously or variably at a predetermined voltage, with adjustable power, to supply it, on the emitter 9, to an electrical impedance or a resistor. The transmission line 15 can comprise an appropriate electrical wire, or coaxial cable, or electromagnetic guide, able to transmit electrical power from the source 8 to the emitter 9. In particular, it is opportune for the thermal emitter 9 constituted by the heating element to be as point-like as possible relative to the dimensions of the hole into which the locking screw is to be driven. As discussed elsewhere, in this case the propagation of radiation in the tissues is coupled with other phenomena of thermal radiation emission from the tissues, due to their heating, and it is therefore also linked to phenomena of heat transport by thermal conduction, thermal convection, irradiation - direct and otherwise - in the liquid inside the bone, in the tissues and in the bone.

A particular variation of the invention generates a signal 14 outside the bone 4 which is reduced to a physical characteristic produced on the bone 4 itself. In particular, alternatively to what has been discussed heretofore, the source 8 comprises a surgical power laser and the emitter 9 comprises an optical focusing system coupled to the transmission line 15, which, when the emitter 9 is in the working position, directs and focuses the laser beam on a part of bone tissue in correspondence with the axis 6a of the predetermined hole 5a. The signal 14 is hence defined by the perforation of the bone effected with the laser beam. The apparatus further comprises protection screens 20, able to be positioned in removable fashion around the fractured part to protect the operator from the action of the laser beam. The direct use of a surgical laser to generate a physical (not optical) signal, i.e. a perforation, leads to safety problems: it is a Class 4 power laser. In particular, the surgical laser acts in this case from the interior of the nail 1 outwardly, being dangerous also in the operating room, even if defocused by the diffusive effect of the bone tissue. Once the bone is holed, the laser beam propagates free in the operating room. This is why the protection screens 20 around the operated part (leg, arm, etc.) are necessary. Therefore, it is also obviously necessary to insert and position the emitter 9 blindly, unless one provides an auxiliary light source that illuminates the predetermined hole 5a from the interior, in ways already discussed for the previous embodiments, and that acts with the surgical laser off (which is complicated, however). Thus, in this variation it is generally also necessary to provide the mechanical systems for exactly guiding, positioning and orienting the emitter 9 in correspondence with the predetermined hole 5a discussed above for similar systems.

A surgical laser can be CO₂ (emission wavelength of about 10 micrometres) or neodymium (emission wavelength of about 1 micrometre). It complicates the system for the reasons described above and because it makes it necessary not only to collimate, but also to focus the beam on the bone. The system can be costly.

On the contrary, a common low power He/Ne laser needs only be coupled to its optical fibre. There are, as stated, (low power) semiconductor lasers (with wavelengths also in the red region) already connected in fibres. These systems are far simpler and economical.

The invention achieves important advantages. It considerably improves on the current technique of driving the locking screws, especially the distal ones. It allows totally to eliminate irradiation with dangerous ionising radiation during the nail locking phase. It makes the operation easy and precise. It allows to use components that, in the preferred embodiments, are simple to manufacture or easy to obtain. The purely mechanical part of the apparatus allows to make the implantation of the endomedullary nail easier, quicker and more precise. Surgical and, consequently, anaesthetic times are drastically reduced. A high precision level in the driving of the locking screws (especially the distal ones) can be achieved in a simple fashion. The simplification of the surgical instrumentation determines a consequent reduction in material production, maintenance, sterilisation and storage costs.

The invention thus conceived can be subject to numerous modifications and variations, without thereby departing from the scope of the claims.

## Claims

1. Apparatus for the osteosynthesis of bone fractures by means of locked endomedullary nailing, of the type comprising:
- a tubular nail (1) defining an internal coaxial through channel (2), provided with transverse through holes (5a, 5b, 5c, 5d) whose axis (6a, 6b, 6c, 6d) intersects the axis (7) of the nail (1) and able to be inserted in a medullar channel (3) of a bone (4);
- a device for locating the axis (6a) of a predetermined hole (5a) selected among said holes (5a, 5b, 5c, 5d), along which the bone (4) is to be drilled to drive a corresponding screw for locking the nail (1) on the bone (4);
**wherein** the locating device comprises:
- a source (8) of electromagnetic power;
- an emitter (9) of the electromagnetic power in the form of non ionising electromagnetic radiation, able to be inserted inside the nail (1) along the internal channel (2) from a proximal end (10) of the nail (1) at least to a working position, located on the axis (6a) of the predetermined hole (5a), in which at least part of the non ionising electromagnetic radiation is directed from the emitter (9), through the predetermined hole (5a), on an inner superficial portion (11) of the cortex (12) of the bone (4) corresponding to the axis (6a) of the predetermined hole (5a) and generates, beyond an outer superficial portion (13) of the cortex (12) of the bone (4), also corresponding to the axis (6a) of the predetermined hole (5a), a signal (14) detectable from the exterior having an intensity distribution with its centroid in correspondence with the axis (6a) of the predetermined hole (5a);
- a line (15) for transmitting the electromagnetic power from the source (8) to the emitter (9); **characterized in that** the non ionising electromagnetic radiation egressing from the emitter (9) has its spectrum distributed on a predetermined interval of wavelenghts selected in the range that goes from near ultraviolet up to and including the mid and the thermal infrared.

2. Apparatus as claimed in claim 1, **characterised in that** the centroid of the intensity distribution of the signal (14) detectable from the exterior coincides with an intensity peak (140).

3. Apparatus as claimed in claim 1, **characterised in that** the electromagnetic radiation egressing from the emitter (9) is visible light.

4. Apparatus as claimed in claim 3, **characterised in that** the wavelengths belonging to the predetermined interval are comprised between 600 nanometres and 700 nanometres.

5. Apparatus as claimed in claim 1, **characterised in that** the emitter (9) comprises an optical collimation system (16) that collimates the electromagnetic radiation in a beam within a predetermined solid angle (17) centred on the optical system (16), the solid angle (17) being coaxial with the predetermined hole (5a) when the emitter (9) is in the working position.

6. Apparatus as claimed in claim 3 or 4, **characterised in that** the emitter (9) comprises an optical collimation system (16) that collimates the electromagnetic radiation in a beam within a predetermined solid angle (17) centred on the optical system (16), the solid angle (17) being coaxial with the predetermined hole (5a) when the emitter (9) is in the working position.

7. Apparatus as claimed in any of the claims 1 through 6, **characterised in that** the source (8) directly generates the non ionising electromagnetic radiation, the transmission line (15) comprising a waveguide for carrying the electromagnetic radiation to the emitter (9).

8. Apparatus as claimed in claim 7, **characterised in that** the waveguide comprises an optical fibre.

9. Apparatus as claimed in claim 5 or 6, **characterised in that** the source (8) directly generates the non ionising electromagnetic radiation, **in that** the transmission line (15) comprises a waveguide for carrying the electromagnetic radiation to the emitter (9) and **in that** the optical system (16), coupled to the waveguide, comprises a collimating lens (18) and a deflector (19) that deflects the radiation coming from the waveguide in the solid angle (17).

10. Apparatus as claimed in claim 1, **characterised in that** the source (8) comprises an electrical power generator, the emitter (9) comprises a heating element and the transmission line (15) comprises an electrical wire connecting the heating element to the electrical power generator, the heating element emitting, when excited by the electrical power, electromagnetic radiation at wavelengths in the thermal infrared region.

11. Apparatus as claimed in claim 1, **characterised in that** the source (8) comprises a surgical power laser and the emitter (9) comprises a focusing optical system coupled with the transmission line (15), which, when the emitter (9) is in the working position, directs and focuses the laser beam on a part of bone tissue in correspondence with the axis (6a) of the predetermined hole (5a), the signal (14) being defined by the perforation of the bone tissue operated with the laser beam, the apparatus further comprising protecting screens (20), able to be positioned in removable fashion around the fractured part to protect the operator from the action of the laser beam.

12. Apparatus as claimed in claim 3 or 4 or 6, **characterised in that** centroid of the intensity distribution of the signal (14) detectable from the exterior coincides with an intensity peak (140) and **in that** the apparatus further comprises a contrast enhancement device (21), which can be positioned on the axis (6a) of the predetermined hole (5a) externally to the bone (4) and acts on the portion of radiation transmitted through the bone tissues enhancing the contrast between the intensity peak (140) and a peripheral part (141) of the signal (14) and improving the resolution in the identification of the position of the axis (6a) of the predetermined hole (5a).

13. Apparatus as claimed in claim 12, **characterised in that** the contrast enhancement device (21) comprises an attenuating filter (22) for attenuating the intensity of the radiation.

14. Apparatus as claimed in claim 13, **characterised in that** the attenuating filter (22) comprises an attenuating element (220) of the type with "neutral density" in the visible range.

15. Apparatus as claimed in claim 14, **characterised in that** the attenuating filter (22) comprises an attenuating element (220) that attenuates the transmission radiation whose wavelength is less than 600 nm.

16. Apparatus as claimed in claim 15, **characterised in that** the transmission spectrum of the attenuating element (220) has a maximum for wavelengths between 600 nm and 700 nm.

17. Apparatus as claimed in any of the claims 13 through 16, **characterised in that** the attenuating filter (22) has an adjustable attenuation factor.

18. Apparatus as claimed in claim 13, **characterised in that** from the emitter (9) egresses electromagnetic radiation with a predetermined polarisation state and **in that** the attenuating filter (22) comprises a polariser element to attenuate the portion of radiation transmitted by the bone tissues not having the polarisation state of the radiation originally egressing from the emitter (9).

19. Apparatus as claimed in any of the claims 1 through 3 or as claimed in claim 5 or 10, **characterised in that** it comprises a detector (24) of the signal (14) and a related image converter, able to be positioned externally to the bone (4) for identifying the centroid of the intensity distribution of the signal (14) and visualising its position.

20. Apparatus as claimed in any of the previous claims, **characterised in that** it comprises automatic positioning means for automatically positioning the emitter (9) at the height of the plane defined by the axis 6a of the predetermined hole 5a and by the axis 7 of the nail 1, acting at least when the emitter (9), inserted in the internal channel (2), is in proximity to the working position.

21. Apparatus as claimed in claim 20, **characterised in that** the automatic positioning means comprise means for centring the emitter (9) on the axis (7) of the nail (1) acting at least when the emitter (9), inserted in the internal channel (2) is in proximity to the working position.

22. Apparatus as claimed in claim 21, **characterised in that** the means for centring the emitter (9) of the axis (7) of the nail (1) comprise at least a widening (27) of the diameter of a sheath of the transmission line (15) at least in proximity to the emitter (9).

23. Apparatus as claimed in claim 22 **characterised in that** the widening (27) extends to enclose the emitter (9) with an element (28) transparent to the electromagnetic radiation egressing from the emitter (9) itself at least in the direction of the predetermined hole (5a).

24. Apparatus as claimed in claim 21, **characterised in that** the means for centring the emitter (9) on the axis of the nail (1) comprise, at least in proximity to the predetermined hole (5a), a narrowing (29) of the internal channel (2) around the axis (7) of the nail (1).

25. Apparatus as claimed in claim 21, **characterised in that** the means for centring the emitter (9) on the axis of the nail (1) comprise at least two straps (30) oriented radially relative to the transmission line (15) and positioned along it in proximity to the emitter (9).

26. Apparatus as claimed in any of the previous claims 20 through 25, **characterised in that** it comprises an arrest reference (25) for arresting the insertion of the emitter (9) in the internal channel (2) when the emitter (9) reaches a working position.

27. Apparatus as claimed in claim 26, **characterised in that** the arrest reference (25) comprises elements (33) for stopping the motion of the transmission line (15) in the nail (1) when the emitter (9) reaches the working position, acting between the transmission line (15) and the wall of the internal channel (2) and able to be elastically disengaged.

28. Apparatus as claimed in claim 26, **characterised in that** the arrest reference (25) comprises an abutment element (26), able to abut the proximal end (10) of the nail (1) or an extension (100) thereof and able to be positioned along the transmission line (15) in a position of arrest of the insertion of the transmission line (15) in the internal channel (2) corresponding to the working position of the emitter (9).

29. Apparatus as claimed in claim 28, **characterised in that** the terminal segment of the transmission line (15) destined to be inserted in the internal channel (2) is enclosed by a substantially rigid sheath and **in that** the abutment element (26) and the proximal end (10) of the nail (1) or its extension (100) comprise mutual engagement means for the certain orientation of the emitter (9) with an axis of emission (90) thereof parallel to the axis (6a) of the predetermined hole (5a).

30. Apparatus as claimed in any of the previous claims 20 through 28, **characterised in that** it comprises means for the certain orientation of the emitter (9) with an axis of emission (90) thereof parallel to the axis (6a) of the predetermined hole (5a) at least in proximity thereto.

31. Apparatus as claimed in claim 30, **characterised in that** the means for the certain orientation of the emitter (9) comprise means (34) of mutual interference between the terminal segment of the transmission line (15), destined to the be inserted in the internal channel (2), and the wall of the internal channel (2), which determine the orientation of the emitter (9) allowing the transmission line (15) to slide in the internal channel (2).

32. Apparatus as claimed in claim 31, **characterised in that** the means (34) of mutual interference means comprise a fin (35) positioned on the transmission line (15) in proximity to the emitter (9) and able to be inserted in sliding fashion at least with one of its ends in a corresponding guiding groove (36) obtained on the surface of the internal channel (2).

33. Apparatus as claimed in any of the previous claims 20 through 32, **characterised in that** at least the terminal segment of the transmission line (15) destined to be inserted in the internal channel (2) comprises a support element with high torsion constant.

34. Apparatus as claimed in any of the previous claims 20 through 32, **characterised in that** at least the terminal segment of the transmission line (15) destined to be inserted in the internal channel (2) is enclosed by a substantially rigid sheath.

35. Apparatus as claimed in any of the previous claims, **characterised in that** it comprises a fastening pin (31) for fastening a tubular hand piece (32) to the nail (1), the fastening pin (31) being tubular, coaxial to the nail (1) and with its inner diameter corresponding to the diameter of the internal channel (2) of the nail (1).

36. Apparatus as claimed in any of the previous claims, **characterised in that** the nail (1) comprises emitters (9), already appropriately positioned and aligned at least in front of corresponding distal holes (5a, 5b), and corresponding segments of transmission line (15), also integrated in the nail (1) and provided, at an opposite end relative to the emitters (9), with connectors to corresponding extensions of the transmission line (15) through to the source (8).

## Patentansprüche

1. Vorrichtung für die Osteosynthese von Knochenbrüchen mittels Markhöhlennagelung, vom Typ enthaltend:
- einen rohrförmigen Nagel (1), der einen internen, koaxialen durchgehenden Kanal (2) beschreibt, versehen mit querverlaufenden durchgehenden Bohrungen (5a, 5b, 5c, 5d), deren Achsen (6a, 6b, 6c, 6d) die Achse (7) des Nagels (1) schneiden, und der in einen Markkanal (3) eines Knochens (4) eingesetzt werden kann;
- eine Vorrichtung zur Lokalisierung der Achse (6a) einer festgelegten Bohrung (5a), gewählt unter den genannten Bohrungen (5a, 5b, 5c, 5d), entlang welcher der Knochen (4) gebohrt werden soll, um eine entsprechende Schraube zum Befestigen des Nagels (1) an dem Knochen (4) einzusetzen;
bei welcher die Lokalisiervorrichtung enthält:
- eine Quelle (8) elektromagnetischer Leistung;
- einen Emitter (9) der elektromagnetischen Leistung in Form von nicht ionisierender elektromagnetischer Strahlung, in der Lage, in das Innere des Nagels entlang dem internen Kanal (2) von einem ungefähren Ende (10) des Nagels (1) bis wenigstens zu einer Arbeitsposition eingesetzt zu werden, angeordnet an der Achse (6a) der festgelegten Bohrung (5a), wobei wenigstens ein Teil der nicht ionisierenden Strahlung von dem Emitter (9) durch die festgelegte Bohrung (5a) auf einen inneren Oberflächenabschnitt (11) der Haut (12) des Knochens (12) gerichtet wird, entsprechend der Achse (6a) der festgelegten Bohrung (5a), und über einen äusseren Oberflächenabschnitt (13) der Haut (12) des Knochens (4) hinaus, welcher ebenfalls der Achse (6a) der festgelegten Bohrung (5a) entspricht, ein Signal (14) erzeugt, das von aussen her zu erfassen ist und eine Intensitätsverteilung mit Schwerpunkt entsprechend der Achse (6a) der festgelegten Bohrung (5a) hat;
- einen Leiter (15) zum Übertragen der elektromagnetischen Leistung von der Quelle (8) an den Emitter (9); **dadurch gekennzeichnet, dass** die aus dem Emitter (9) austretende nicht ionisierende Strahlung ihr Spektrum auf einem vorgegebenen Wellenlängenintervall verteilt hat, gewählt in dem Bereich, der von dicht an Ultraviolett bis einschliesslich dem mittleren und dem thermischen Infrarot reicht.

2. Vorrichtung nach Patentanspruch 1, **dadurch gekennzeichnet, dass** der Schwerpunkt der Intensitätsverteilung des Signals (14), der von aussen her erfassbar ist, mit einer Intensitätsspitze (140) übereinstimmt.

3. Vorrichtung nach Patentanspruch 1, **dadurch gekennzeichnet, dass** die aus dem Emitter (9) austretende elektromagnetische Strahlung sichtbares Licht ist.

4. Vorrichtung nach Patentanspruch 3, **dadurch gekennzeichnet, dass** die Wellenlängen, die zu dem vorgegebenen Intervall gehören, zwischen 600 Nanometern und 700 Nanometern enthalten sind.

5. Vorrichtung nach Patentanspruch 1, **dadurch gekennzeichnet, dass** der Emitter (9) ein optisches Einstellsystem (16) enthält, welches die elektromagnetische Strahlung in einem Bündel innerhalb eines vorgegebenen Raumwinkels (17) richtet, der auf das optische System (16) zentriert ist, wobei der Raumwinkel (17) koaxial mit der festgelegten Bohrung (5a) ist, wenn der Emitter (9) sich in der Arbeitsposition befindet.

6. Vorrichtung nach Patentanspruch 3 oder 4, **dadurch gekennzeichnet, dass** der Emitter (9) ein optisches Bündelungssystem (16) enthält, welches die elektromagnetische Strahlung in einem Bündel innerhalb eines vorgegebenen Raumwinkels (17) richtet, der auf das optische System (16) zentriert ist, wobei der Raumwinkel (17) koaxial mit der festgelegten Bohrung (5a) ist, wenn der Emitter (9) sich in der Arbeitsposition befindet.

7. Vorrichtung nach einem beliebigen der Patentansprüche von 1 bis 6, **dadurch gekennzeichnet, dass** die Quelle (8) direkt die nicht ionisierende elektromagnetische Strahlung erzeugt, wobei der Übertragungsleiter (15) einen Wellenleiter zum Übertragen der elektromagnetischen Strahlung an den Emitter (9) enthält.

8. Vorrichtung nach Patentanspruch 7, **dadurch gekennzeichnet, dass** der Wellenleiter eine optische Faser enthält.

9. Vorrichtung nach Patentanspruch 5 oder 6, **dadurch gekennzeichnet, dass** die Quelle (8) direkt die nicht ionisierende elektromagnetische Strahlung erzeugt, **dadurch**, dass der Übertragungsleiter (15) einen Wellenleiter zum Übertragen der elektromagnetischen Strahlung an den Emitter (9) enthält, und **dadurch**, dass das an den Wellenleiter angeschlossene optische System (16) eine Bündelungslinse (18) und einen Ableiter (19) enthält, der die von dem Wellenleiter kommende Strahlung in den Raumwinkel (17) ableitet.

10. Vorrichtung nach Patentanspruch 1, **dadurch gekennzeichnet, dass** die Quelle (8) einen elektrischen Leistungsgenerator enthält, der Emitter (9) ein Heizelement und der Übertragungsleiter (15) einen elektrischen Draht, welcher das Heizelement mit dem elektrischen Leistungsgenerator verbindet, wobei das Heizelement, wenn es durch die elektrische Leistung erregt ist, elektromagnetische Strahlung bei Wellenlängen im thermischen Infrarotbereich aussendet.

11. Vorrichtung nach Patentanspruch 1, **dadurch gekennzeichnet, dass** die Quelle (8) einen chirurgischen Leistungslaser enthält und der Emitter (9) ein fokussierendes optisches System, verbunden mit dem Übertragungsleiter (15), welcher, wenn sich der Emitter (9) in der Arbeitsposition befindet, das Laserbündel auf einen Teil des Knochengewebes entsprechend der Achse (6a) der festgelegten Bohrung (5a) richtet und auf diesem fokussiert, wobei das Signal (14) durch die Perforation des Knochengewebes beschrieben ist, ausgeführt mit dem Laserbündel, wobei die Vorrichtung ausserdem Schutzschirme (20) enthält, die auf entfernbare Weise rund um den gebrochenen Teil angeordnet werden können, um den Operateur vor die Wirkung des Laserbündels zu schützen.

12. Vorrichtung nach Patentanspruch 3 oder 4 oder 6, **dadurch gekennzeichnet, dass** der Schwerpunkt der Intensitätsverteilung des Signals (14), erfassbar von aussen her, mit einer Intensitätsspitze (140) übereinstimmt, und **dadurch**, dass die Vorrichtung ausserdem eine Kontrasterhöhungsvorrichtung (21) enthält, die an der Achse (6a) der festgelegten Bohrung (5a) ausserhalb des Knochens (4) angeordnet werden kann und auf den Abschnitt der durch das Knochengewebe übertragenen Strahlung wirkt, wobei der Kontrast zwischen der Intensitätsspitze (140) und dem umgebenden Teil (141) des Signals (14) erhöht und die Auflösung in der Erkennung der Position der Achse (6a) der festgelegten Bohrung (5a) verbessert wird.

13. Vorrichtung nach Patentanspruch 12, **dadurch gekennzeichnet, dass** die Kontrasterhöhungsvorrichtung (21) einen Abschwächungsfilter (22) zum Abschwächen der Strahlungsintensität enthält.

14. Vorrichtung nach Patentanspruch 13, **dadurch gekennzeichnet, dass** der Abschwächungsfilter (22) ein Abschwächungselement (220) vom "neutralgrauen" Typ im sichtbaren Bereich enthält.

15. Vorrichtung nach Patentanspruch 14, **dadurch gekennzeichnet, dass** der Abschwächungsfilter (22) ein Abschwächungselement (220) enthält, welches die übertragene Strahlung abschwächt, deren Wellenlänge geringer ist als 600 nm.

16. Vorrichtung nach Patentanspruch 15, **dadurch gekennzeichnet, dass** das Übertragungsspektrum des Abschwächelementes (220) ein Maximum für Wellenlängen zwischen 600 nm und 700 nm hat.

17. Vorrichtung nach einem jeden der Patentansprüche von 13 bis 16, **dadurch gekennzeichnet, dass** der Abschwächungsfilter (22) einen regelbaren Abschwächungsfaktor hat.

18. Vorrichtung nach Patentanspruch 13, **dadurch gekennzeichnet, dass** aus dem Emitter (9) eine elektromagnetische Strahlung mit einem bestimmten Polarisationszustand austritt, und dass der Abschwächungsfilter (22) ein Polarisationselement enthält, um den Abschnitt der durch das Knochengewebe übertragenen Strahlung abzuschwächen, der nicht den Polarisationszustand der ursprünglich aus dem Emitter (9) austretenden Strahlung hat.

19. Vorrichtung nach einem jeden der Patentansprüche von 1 bis 3 oder nach dem Patentanspruch 5 oder 10, **dadurch gekennzeichnet, dass** sie einen Detektor (24) des Signals (14) und einen entsprechenden Bildumwandler enthält, in der Lage, ausserhalb des Knochens (4) angeordnet zu werden, um den Schwerpunkt der Intensitätsverteilung des Signals (14) zu erfassen und dessen Position sichtbar zu machen.

20. Vorrichtung nach einem beliebigen der vorstehenden Patentansprüche, **dadurch gekennzeichnet, dass** sie automatische Positioniermittel zur automatischen Positionierung des Emitters (9) auf der Höhe der Ebene enthält, die durch die Achse (6a) der festgelegten Bohrung (5a) festgelegt ist, sowie durch die Achse (7) des Nagels (1), welche wenigstens tätig sind, wenn der in den internen Kanal (2) eingesetzte Emitter (9) sich in der Nähe der Arbeitsposition befindet.

21. Vorrichtung nach Patentanspruch 20, **dadurch gekennzeichnet, dass** die automatischen Positioniermittel Mittel zum Zentrieren des Emitters (9) auf der Achse (7) des Nagels (1) enthalten, die wenigstens tätig sind, wenn der in den internen Kanal (2) eingesetzte Emitter (9) sich in der Nähe der Arbeitsposition befindet.

22. Vorrichtung nach Patentanspruch 21, **dadurch gekennzeichnet, dass** die Mittel zum Zentrieren des Emitters (9) auf der Achse (7) des Nagels (1) mindestens eine Erweiterung (27) von einem Durchmesser einer Hülle des Übertragungskanals (15) enthalten, wenigstens in der Nähe des Emitters (9).

23. Vorrichtung nach Patentanspruch 22, **dadurch gekennzeichnet, dass** die Erweiterung (27) sich ausdehnt, um den Emitter (9) mit einem Element (28) einzuschliessen, dass durchlässig für die aus dem Emitter (9) selbst austretende elektromagnetische Strahlung ist, und zwar wenigstens in der Richtung der festgelegten Bohrung (5a).

24. Vorrichtung nach Patentanspruch 21, **dadurch gekennzeichnet, dass** die Mittel zum Zentrieren des Emitters (9) auf der Achse des Nagels (1) wenigstens in der Nähe der festgelegten Bohrung (5a) eine Verengung (29) des internen Kanals (2) rund um die Achse (7) des Nagels (1) enthalten.

25. Vorrichtung nach Patentanspruch 21, **dadurch gekennzeichnet, dass** die Mittel zum Zentrieren des Emitters (9) auf der Achse des Nagels (1) wenigstens zwei Stege (30) enthalten, die radial im Verhältnis zu dem Übertragungskanal (15) orientiert und entlang diesem in der Nähe des Emitters (9) positioniert sind.

26. Vorrichtung nach einem beliebigen der vorstehenden Patentansprüche von 20 bis 25, **dadurch gekennzeichnet, dass** sie einen Bezugsanschlag (25) zum Anhalten des Einschiebens des Emitters (9) in den internen Kanal (2) enthält, wenn der Emitter (9) eine Arbeitsposition erreicht.

27. Vorrichtung nach Patentanspruch 26, **dadurch gekennzeichnet, dass** der Bezugsanschlag (25) Elemente (33) zum Anhalten der Bewegung des Übertragungsleiters (15) in dem Nagel (1) enthält, wenn der Emitter (9) die Arbeitsposition erreicht, die zwischen dem Übertragungsleiter (15) und der Wand des internen Kanals (2) wirken und die elastisch freigegeben werden können.

28. Vorrichtung nach Patentanspruch 26, **dadurch gekennzeichnet, dass** der Bezugsanschlag (25) ein Anschlagelement (26) aufweist, in der Lage, auf das proximale Ende (10) des Nagels (1) oder eine Verlängerung (100) desselben zu treffen, und in der Lage, entlang dem Übertragungsleiter (15) in einer Anschlagposition des Einschiebens des Übertragungsleiters (15) in den internen Kanal (2) entsprechend der Arbeitsposition des Emitters (9) angeordnet zu werden.

29. Vorrichtung nach Patentanspruch 28, **dadurch gekennzeichnet, dass** der Endabschnitt des Übertragungsleiters (15), der dazu bestimmt ist, in den internen Kanal (2) eingesetzt zu werden, in einer im wesentlichen starren Hülle eingeschlossen ist, und **dadurch**, dass das Anschlagelement (26) und das proximale Ende (10) des Nagels (1) oder dessen Verlängerung (100) gegenseitige Eingriffsmittel enthalten, und zwar für die sichere Orientierung des Emitters (9) mit einer Emissionsachse (90) desselben parallel zu der Achse (6a) der festgelegten Bohrung (5a).

30. Vorrichtung nach einem beliebigen der vorstehenden Patentansprüche von 20 bis 28, **dadurch gekennzeichnet, dass** sie Mittel zur sicheren Orientierung des Emitters (9) enthält, mit einer Emissionsachse (90) desselben parallel zu der Achse (6a) der festgelegten Bohrung (5a), wenigstens in der Nähe von letzterer.

31. Vorrichtung nach Patentanspruch 30, **dadurch gekennzeichnet, dass** die Mittel zur sicheren Orientierung des Emitters (9) Mittel (34) der gegenseitigen Interferenz zwischen dem Endabschnitt des Übertragungsleiters (15), der zum Einsetzen in den internen Kanal (2) bestimmt ist, und der Wand des internen Kanals (2) enthalten, welche die Orientierung des Emitters (9) bestimmen und es dem Übertragungsleiter (15) ermöglichen, in dem internen Kanal (2) zu gleiten.

32. Vorrichtung nach Patentanspruch 31, **dadurch gekennzeichnet, dass** die Mittel (34) der gegenseitigen Interferenz eine Flosse (35) enthalten, positioniert an dem Übertragungsleiter (15) in der Nähe des Emitters (9) und geeignet, in verschiebbarer Weise wenigstens mit einem ihrer Enden in eine entsprechende Führungsrille (36) eingesetzt zu werden, die an der Oberfläche des internen Kanals (2) erhalten ist.

33. Vorrichtung nach einem beliebigen der vorstehenden Patentansprüche von 20 bis 32, **dadurch gekennzeichnet, dass** wenigstens der Endabschnitt des Übertragungsleiters (15), der dazu bestimmt ist, in den internen Kanal (2) eingesetzt zu werden, ein Halteelement mit hoher Verdrehungskonstante enthält.

34. Vorrichtung nach einem beliebigen der vorstehenden Patentansprüche von 20 bis 32, **dadurch gekennzeichnet, dass** wenigstens der Endabschnitt des Übertragungsleiters (15), der dazu bestimmt ist, in den internen Kanal (2) eingesetzt zu werden, im wesentlichen in einer starren Hülle eingeschlossen ist.

35. Vorrichtung nach einem beliebigen der vorstehenden Patentansprüche, **dadurch gekennzeichnet, dass** sie einen Befestigungszapfen (31) zum Anbringen eines rohrförmigen Handstückes (32) an dem Nagel (1) enthält, wobei der Befestigungszapfen (31) rohrförmig ist, koaxial zu dem Nagel (1) verläuft und mit seinem Innendurchmesser dem Durchmesser den internen Kanals (2) des Nagels (1) entspricht.

36. Vorrichtung nach einem beliebigen der vorstehenden Patentansprüche, **dadurch gekennzeichnet, dass** der Nagel (1) Emitter (9) enthält, die bereits geeignet positioniert und wenigstens zu entsprechenden distalen Bohrungen (5a, 5b) ausgerichtet sind, und die Abschnitten des Übertragungsleiters (15) entsprechen, die ebenfalls in den Nagel (1) integriert und an einem im Verhältnis zu den Emittern (9) entgegengesetzten Ende mit Verbindungsteilen zu entsprechenden Verlängerungen des Übertragungsleiters (15) bis zu der Quelle (8) versehen sind.

## Revendications

1. Appareil pour l'ostéosynthèse de fragments osseux par clou endomédullaire, du type comprenant:
- un clou tubulaire (1) définissant un canal interne passant coaxial (2), pourvu d'orifices passants transversaux (5a, 5b, 5c, 5d) dont les axes (6a, 6b, 6c, 6d) croisent l'axe (7) du clou (1), et pouvant être inséré dans un canal médullaire (3) d'un os (4);
- un dispositif de localisation de l'axe (6a) d'un orifice (5a) prédéterminé sélectionné parmi lesdits orifices (5a, 5b, 5c, 5d), le long duquel l'os (4) doit être perforé pour guider une vis correspondante de blocage du clou (1) sur l'os (4);
dans lequel le dispositif de localisation comprend:
- une source (8) de puissance électromagnétique;
- un émetteur (9) de puissance électromagnétique sous forme d'une radiation électromagnétique non ionisante, pouvant être inséré à l'intérieur du clou (1) le long du canal interne (2) à partir d'une extrémité proximale (10) du clou (1) au moins dans une position de travail, située sur l'axe (6a) de l'orifice prédéterminé (5a), dans lequel au moins une partie de la radiation électromagnétique non ionisante est dirigée à partir de l'émetteur (9), au travers de l'orifice prédéterminé (5a), sur une partie superficielle interne (11) du cortex (12) de l'os (4) correspondant à l'axe (6a) de l'orifice prédéterminé (5a) et génère, au-delà d'une partie superficielle externe (13) du cortex (12) de l'os (4), correspondant également à l'axe (6a) de l'orifice prédéterminé (5a), un signal (14) détectable de l'extérieur ayant une distribution de l'intensité avec son barycentre en correspondance de l'axe (6a) de l'orifice prédéterminé (5a);
- une ligne (15) de transmission de la puissance électromagnétique de la source (8) à l'émetteur (9);
**caractérisé en ce que** la radiation électromagnétique non ionisante émanant de l'émetteur (9) présente un spectre distribué sur un intervalle prédéterminé de longueurs d'onde étendu entre les ultraviolets jusqu'aux infrarouges moyens et thermiques en comprenant ces derniers.

2. Appareil selon la revendication 1, **caractérisé en ce que** le barycentre de la distribution d'intensité du signal (14) détectable depuis l'extérieur coïncide avec un pic d'intensité (140).

3. Appareil selon la revendication 1, **caractérisé en ce que** la radiation électromagnétique émanant de l'émetteur (9) est une lumière visible.

4. Appareil selon la revendication 3, **caractérisé en ce que** les longueurs d'onde faisant partie de l'intervalle prédéterminé sont comprises entre 600 nanomètres et 700 nanomètres.

5. Appareil selon la revendication 1, **caractérisé en ce que** l'émetteur (9) comprend un système optique de collimation (16) qui collimate la radiation électromagnétique dans un faisceau à l'intérieur d'un angle solide (17) centré sur le système optique (16), l'angle solide (17) étant coaxial à l'orifice prédéterminé (5a) lorsque l'émetteur (9) se trouve dans la position de travail.

6. Appareil selon la revendication 3 ou 4, **caractérisé en ce que** l'émetteur (9) comprend un système optique de collimation (16) qui collimate la radiation électromagnétique dans un faisceau à l'intérieur d'un angle solide prédéterminé (17) centré sur le système optique (16), l'angle solide (17) étant coaxial à l'orifice prédéterminé (5a) lorsque l'émetteur (9) se trouve dans la position de travail.

7. Appareil selon n'importe laquelle des revendications 1 à 6, **caractérisé en ce que** la source (8) génère directement la radiation électromagnétique non ionisante, la ligne de transmission (15) comprenant un guide d'onde pour le transport de la radiation électromagnétique vers l'émetteur (9).

8. Appareil selon la revendication 7, **caractérisé en ce que** le guide d'onde comprend une fibre optique.

9. Appareil selon la revendication 5 ou 6, **caractérisé en ce que** la source (8) génère directement la radiation électromagnétique non ionisante, **en ce que** la ligne de transmission (15) comprend un guide d'onde pour le transport de la radiation électromagnétique vers l'émetteur (9) et **en ce que** le système optique (16), accouplé au guide d'onde, comprend une lentille de collimation (18) et un déflecteur (19) qui défléchit la radiation provenant du guide d'onde dans l'angle solide (17).

10. Appareil selon la revendication 1, **caractérisé en ce que** la source (8) comprend un générateur de puissance électrique, l'émetteur (9) comprend un élément de chauffe et la ligne de transmission (15) comprend un câble électrique reliant l'élément de chauffe au générateur de puissance électrique, l'élément chauffant émettant, lorsqu'il est excité par le courant électrique, une radiation électromagnétique présentant une longueur d'onde qui se situe dans la zone des infrarouges thermiques.

11. Appareil selon la revendication 1, **caractérisé en ce que** la source (8) comprend un laser chirurgical de puissance et l'émetteur (9) comprend un système optique de focalisation accouplé à la ligne de transmission (15), lequel, lorsque l'émetteur (9) se trouve dans la position de travail, dirige et focalise le faisceau laser sur une partie de tissu osseux en correspondance de l'axe (6a) de l'orifice prédéterminé (5a), le signal (14) étant défini par la perforation du tissu osseux opérée par un faisceau laser, l'appareil comprenant en outre des écrans de protection (20), pouvant être positionnés de manière amovible autour de la partie fracturée pour protéger l'opérateur de l'action du faisceau laser.

12. Appareil selon la revendication 3 ou 4 ou 6, **caractérisé en ce que** le barycentre de la distribution d'intensité du signal (14) détectable depuis l'extérieur coïncide avec un pic d'intensité (140) et **en ce que** l'appareil comprend de plus un dispositif d'incrémentation du contraste (21), lequel peut être positionné sur l'axe (6a) de l'orifice prédéterminé (5a) extérieurement à l'os (4) et agit sur la partie de radiation transmise au travers des tissus osseux augmentant le contraste entre le pic d'intensité (140) et une partie périphérique (141) du signal (14) et améliorant la résolution dans l'identification de la position de l'axe (6a) de l'orifice prédéterminé (5a).

13. Appareil selon la revendication 12, **caractérisé en ce que** le dispositif d'incrémentation du contraste (21) comprend un filtre atténuant (22) pour atténuer l'intensité de la radiation.

14. Appareil selon la revendication 13, **caractérisé en ce que** le filtre atténuant (22) comprend un élément atténuant (220) du type "neutral density" dans le champ du visible.

15. Appareil selon la revendication 14, **caractérisé en ce que** le filtre atténuant (22) comprend un élément atténuant (220) qui atténue la radiation de transmission dont la longueur d'onde est inférieure à 600 nanomètres.

16. Appareil selon la revendication 15, **caractérisé en ce que** le spectre de transmission de l'élément atténuant (220) présente un maximum par longueurs d'ondes entre 600 nanomètres et 700 nanomètres.

17. Appareil selon n'importe laquelle des revendications 13 à 16, **caractérisé en ce que** le filtre atténuant (22) présente un facteur d'atténuation réglable.

18. Appareil selon la revendication 13, **caractérisé en ce qu'**émerge de l'émetteur (9) une radiation électromagnétique avec un état de polarisation prédéterminé et **en ce que** le filtre atténuant (22) comprend un élément polarisateur pour atténuer la partie de radiation transmise par les tissus osseux n'ayant pas l'état de polarisation de la radiation émergeant originalement de l'émetteur (9).

19. Appareil selon n'importe laquelle des revendications 1 à 3 ou selon la revendication 5 ou 10, **caractérisé en ce qu'**il comprend un détecteur (24) du signal (14) et un relatif convertisseur d'image, pouvant être positionné extérieurement à l'os (4) pour l'identification du barycentre de la distribution d'intensité du signal (14) et la visualisation de sa position.

20. Appareil selon n'importe laquelle des revendications précédentes, **caractérisé en ce qu'**il comprend des moyens de positionnement automatique pour positionner automatiquement l'émetteur (9) à la hauteur du plan défini par l'axe (6a) de l'orifice prédéterminé (5a) et par l'axe (7) du clou (1), agissant au moins lorsque l'émetteur (9), inséré dans le canal interne (2), se trouve à proximité de la position de travail.

21. Appareil selon la revendication 20, **caractérisé en ce que** les moyens de positionnement automatique comprennent des moyens de centrage de l'émetteur (9) sur l'axe (7) du clou (1) agissant lorsque l'émetteur (9), inséré dans le canal interne (2) se trouve à proximité de la position de travail.

22. Appareil selon la revendication 21, **caractérisé en ce que** les moyens de centrage de l'émetteur (9) de l'axe (7) du clou (1) comprennent au moins un élargissement (27) du diamètre d'une gaine de la ligne de transmission (15) au moins à proximité de l'émetteur (9).

23. Appareil selon la revendication 22, **caractérisé en ce que** l'élargissement (27) s'étend pour renfermer l'émetteur (9) avec un élément (28) transparent à la radiation électromagnétique émergeant de l'émetteur (9) lui-même au moins dans la direction de l'orifice prédéterminé (5a).

24. Appareil selon la revendication 21, **caractérisé en ce que** les moyens de centrage de l'émetteur (9) sur l'axe du clou (1) comprennent, au moins à proximité de l'orifice prédéterminé (5a), un rétrécissement (29) du canal interne (2) autour de l'axe (7) du clou (1).

25. Appareil selon la revendication 21, **caractérisé en ce que** les moyens de centrage de l'émetteur (9) sur l'axe du clou (1) comprennent au moins deux bandes (30) orientées radialement par rapport à la ligne de transmission (15) et positionnées le long de cette dernière à proximité de l'émetteur (9).

26. Appareil selon n'importe laquelle des revendications précédentes 20 à 25, **caractérisé en ce qu'**il comprend une référence d'arrêt (25) de l'insertion de l'émetteur (9) dans le canal interne (2) lorsque l'émetteur (9) atteint une position de travail.

27. Appareil selon la revendication 26, **caractérisé en ce que** la référence d'arrêt (25) comprend des éléments (33) d'arrêt du mouvement de la ligne de transmission (15) dans le clou (1) lorsque l'émetteur (9) atteint la position de travail, agissant entre la ligne de transmission (15) et la paroi du canal interne (2) et pouvant être désengagés de manière élastique.

28. Appareil selon la revendication 26, **caractérisé en ce que** la référence d'arrêt (25) comprend un élément de butée (26), pouvant buter sur l'extrémité proximale (10) du clou (1) ou une extension (100) lui appartenant et pouvant être positionné le long de la ligne de transmission (15) dans une position d'arrêt de l'insertion de la ligne de transmission (15) dans le canal interne (2) correspondant à la position de travail de l'émetteur (9).

29. Appareil selon la revendication 28, **caractérisé en ce que** le segment terminal de la ligne de transmission (15) destiné à être inséré dans le canal interne (2) est renfermé dans une gaine substantiellement rigide et **en ce que** l'élément de butée (26) et l'extrémité proximale (10) du clou (1) ou son extension (100) comprennent des moyens d'engagement mutuel pour l'orientation sûre de l'émetteur (9) avec un axe d'émission (90) lui appartenant parallèle à l'axe (6a) de l'orifice prédéterminé (5a).

30. Appareil selon n'importe laquelle des revendications précédentes 20 à 28, **caractérisé en ce qu'**il comprend des moyens pour l'orientation sûre de l'émetteur (9) avec un axe d'émission (90) lui appartenant parallèle à l'axe (6a) de l'orifice prédéterminé (5a) au moins à proximité de ce dernier.

31. Appareil selon la revendication 30, **caractérisé en ce que** les moyens pour l'orientation sûre de l'émetteur (9) comprennent des moyens (34) d'interférence mutuelle entre le segment terminal de la ligne de transmission (15), destiné à être inséré dans le canal interne (2), et la paroi du canal interne (2), qui détermine l'orientation de l'émetteur (9) permettant à la ligne de transmission (15) de coulisser dans le canal interne (2).

32. Appareil selon la revendication 31, **caractérisé en ce que** les moyens (34) d'interférence mutuelle comprennent une ailette (35) positionnée sur la ligne de transmission (15) à proximité de l'émetteur (9) et pouvant être insérée de manière coulissante au moins avec l'une de ses extrémités dans une correspondante rainure de guidage (36) obtenue sur la surface du canal interne (2).

33. Appareil selon n'importe laquelle des revendications précédentes 20 à 32, **caractérisé en ce qu'**au moins le segment terminal de la ligne de transmission (15) destiné à être inséré dans le canal interne (2) comprend un élément de support présentant une constante de torsion élevée.

34. Appareil selon n'importe laquelle des revendications précédentes 20 à 32, **caractérisé en ce qu'**au moins le segment terminal de la ligne de transmission (15) destiné à être inséré dans le canal interne (2) est renfermé par une gaine substantiellement rigide.

35. Appareil selon n'importe laquelle des revendications précédentes, **caractérisé en ce qu'**il comprend une broche de fixation (31) pour fixer une pièce tubulaire de préhension (32) au clou (2), la broche de fixation (31) étant tubulaire, coaxiale au clou (1) et avec son diamètre interne correspondant au diamètre du canal interne (2) du clou (1).

36. Appareil selon n'importe laquelle des revendications précédentes, **caractérisé en ce que** le clou (1) comprend des émetteurs (9), déjà positionnés et alignés de manière appropriée au moins en face des orifices distaux (5a, 5b) correspondants, et de correspondants segments de ligne de transmission (15), également intégrés dans le clou (1) et pourvus, sur une extrémité opposée relative aux émetteurs (9), de connecteurs à des correspondantes extensions de la ligne de transmission (15) jusqu'à la source (8).
